# EUROPEAN PATENT APPLICATION

(11) **EP 1 417 928 A1**
(43) Date of publication of application: **12.05.2004**
(21) Application number: 02079707.2
(22) Date of filing: 11.11.2002
(51) Int. Cl.: A61B 5/11, G08B 21/22

(54) **Detection system for patient's movements out of a chair or bed**

(71) Applicant: Daza Opti Care, 4708 KP Roosendaal (NL)
(72) Inventor: Danen, Adrianus Wilhemus Marinus, 4708 KP Roosendaal (NL); van der Zande, Willem Marinus, 4793 GX Fijnaart (NL)

(57) **Abstract**

The invention relates to a very inventive total detection system (1) for a resident/patient seated on a chair or lying on a bed, in which this patient will not start to walk around without supervision of a nurse and thus falling accidents with nasty effects occur, in which the mentioned total detection system (1) consists of a cushion or mat (2) provided with a very sensitive pressure sensor (3), which is electrically connected to a control or transmitter box (5) for adjustment and if necessary transmitting signals to a receiver box (6), in which approximately 10 cushions or mats (2) can be read in to one receiver box (6), which can have a common construction, after which a nurse can rush to the patient, so that in the living room falling accidents can be avoided which are, due to fatigue at the end of the day.

## Description

The present invention relates to a Apparatus constructed as a detection system shaped as a flat cushion or mat for placement on, in or underneath a chair's pillow or bed's mattress, in which the mentioned cushion or mat is provided with a mechanical/electronic total system for detecting and transmitting the applied changes in load and status on the mentioned cushion or mat in which the mentioned cushion or mat is mostly placed in a chair or bed of a resident, mostly a patient who is already admissioned in a home or nursing home.

In practice it appears that residents of homes and/or nursing homes, whom have mostly reached the state of patient, must be watched clearly by nurses and attendants or carers, especially in the afternoon. The fatigue of the mentioned patients particularly increases in the afternoon and if they want to leave their chair or bed without supervision or help from personnel, a lot of falling accidents appear to happen. In the afternoon the patients mostly gather in groups in the living room of the nursing home and the attendants/nurses are not present all the time. Then if a patient tries to get up out of the chair, he cannot be stopped by the other patients, or worse, falling accidents cannot be avoided. In practice there appears to be a need, especially in nursery homes, for an information or alarm system for nurses and carers at distant, outside the mentioned living room, to inform them if a patient leaves his chair or bed. Besides, several chairs and beds with patients are present in such a living room, which can be left arbitrary by patients. In short, there is a need for a detection system, which in a central unit of the nurses indicates in which living room or room in general a patient has left his chair or bed, in which the patient's sitting or lying comfort may not be influenced. Besides, the self-correcting ability of the resident or patient must also be anticipated. This means, that only a signal is sent to the central unit of the nurses if the patient has not returned into his chair or bed after a pre-set number of seconds. The distance between the living room and central unit of the nurses can be quite large.

Therefore, there is a need for an answer to this problem and this is possible in the form of a detection system for indicating differences in load which is extended or also provided with a control or transmitting possibility in the mentioned living room and a receiver possibility which is within the nurses reach.

In practice, in the car industry an alarm system is known, which is built in when changes in load take place on the car seat. But the known mentioned systems in the car industry have a number of disadvantages with regard to the fact that these cannot be applied to a chair in a simple way and cannot be removed with a few movements by a non-technical person and furthermore the signal cannot be transmitted over a distance of several tens of metres, in which walls and such can be situated between the rooms. Here, the car is loaded in a certain closed space, in which the signal must be able to be received in another room at a large distance. Also, for use in a nursing home several adjustment possibilities are necessary.

The aim of the present invention concerns to provide such a detection system especially for patient movements out of chairs and beds, with which leaving the chair or bed by the patient can be detected and transmitted in a very effective manner to the nurses room or a mobile nurse and in which the system is durably adjustable and controllable and of which the aforementioned disadvantages of the system built in a car seat are eliminated. Furthermore the system or detection system should be produced in an economical way.

In other words, the aim is to reduce the number of falling accidents and postpone the securing of patients to the chair or bed in nursery homes, as well as for special use in hospitals, in short where someone may not leave his/her chair or bed without supervision, because these persons must be protected against themselves. Furthermore the requirements to reach the aim are formulated as follows:
- The detection method and technology used may not influence the sitting comfort of the chair or the lying comfort of the bed.
- The material used may not cause bedsores.
- The upholstery of the cushion or mat must be made of incontinent material.
- The self-correcting ability of the resident or patient must be anticipated.
- No loose wires on the floor of, for example the living room.
- A signal must be given when the battery for the electronics threatens to run down.
- The nurse should receive a signal if he/she forgets to switch on the detection system.
- The nurses must be able to choose if the buzzer of the detection system for self-correcting ability of the habitant is switched on or off.

These items according to the invention have been worked out in the claims.

Therefore, a Apparatus constructed as a detection system is developed as a flat cushion or mat for placement on, in or underneath a chair's pillow or bed's mattress, and is developed in a very inventive way, characterized in that, the mentioned mechanical/electronic total detection system is constructed of at least one sensor applied in the mentioned cushion or mat, which is mostly applied centrally in the mentioned cushion or mat, which sensor is electrically or electronically coupled with an elsewhere mounted control or transmitter box and which is supplied with electronic circuits for switching on and off of the mentioned total detection system, a reminder routine, an observation system for changes in pressure on the sensor if this is forced by the patient and detection of rising and/or sitting down again or lying down of the patient, the status of the total detection system, the detection of almost running down of the battery and transmitting signals to the receiver box for calling a nurse/carer, and in which the mentioned receiver box, which can be a universal common receiver box, in which the mentioned mechanical/electronic total detection system is energized by a battery with an operating voltage of 3 Volt, in which the content and the exterior of the mentioned cushion or mat can be provided with comfort increasing facilities.

The advantage is an attachable detection system which is very manageable, fast and handy for the nurses, which system can be attached to any chair or bed.

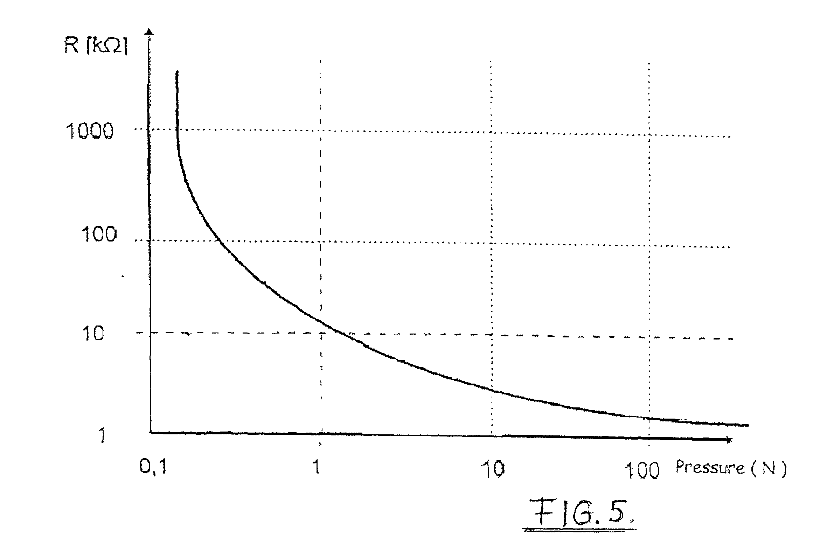

Furthermore the Apparatus constructed as a detection system according to the invention is further developed in such a way, that the mentioned sensor is a pressure sensor of the FSR-sensor type (Force Sensing Resistor), in which the mentioned FSR-sensor is built-up in three layers, being a load-bearing foil with pressed-on FSR-layer, and adhesive layer (spacer) and a load-bearing foil with electrodes, in which this can have the shape of a single element with a diameter of approximately 40 mm, in which the connection between the applied forces in Newton on the sensor and the electronic resistance R in kΩ can be as in figure 5.

The advantages are, that a commonly available, sensitive sensor is used with very small dimensions and which has a very large measuring range. Due to the very small dimensions the patient will not feel anything and will not be bothered.

Furthermore the Apparatus constructed as a detection system according to the invention is developed in such a way, that the set-up and operation of the electronic circuit and/or programming for switching on and off is indicated in figure 6, in which the set-up and operation of the electronic circuit for the reminder routine is indicated in figure 7, in which the set-up and operation of the electronic circuit and/or programming for sitting/lying down and getting up is indicated in figure 8, in which the set-up and operation of the electronic circuit and/or programming for the LED's status is indicated in figure 9, in which the set-up and operation of the electronic circuit and/or programming for battery discharge announcement is indicated in figure 10.

The advantages are that the functions are controlled adequately and that accidentally bumping against the switch by a resident or patient, which mostly lasts less than 2,5 seconds, will not activate the system and that by the several short messages the detection system uses minimum energy and that the status of the detection system is constantly obvious and that transmitting signals to the nurses receivers after a clear fall out of the pressure will only take place in a pre-set amount of time and also that running down of the battery is clearly indicated, which increases the safety.

Furthermore the Apparatus constructed as a detection system according to the invention is further developed in such a way, that the contents of the mentioned cushion or mat with regard to flexibility is made of the material BULTEX, being a durable resilient material, in which the upholstery of the mentioned cushion or mat for increasing comfort with regard to water tightness and antiskid is made of so called incontinent material.

The advantages are an obvious durable comfort and no inner pollution of mentioned cushion or mat, in which it also stays resilient.

Furthermore the Apparatus constructed as a detection system according to the invention is further developed in such a way, that maximally 10 cushions or mats can be read in on the mentioned receiver box, so that in each accommodation a closed surveillance/monitoring system for the resident and/or patient is created.

The advantage is, that the maximum number of chairs or beds present in the living room can all be connected to the mentioned system.

The preferred construction of the invention will be described by way of example, and with reference to the accompanying drawing.

In which:

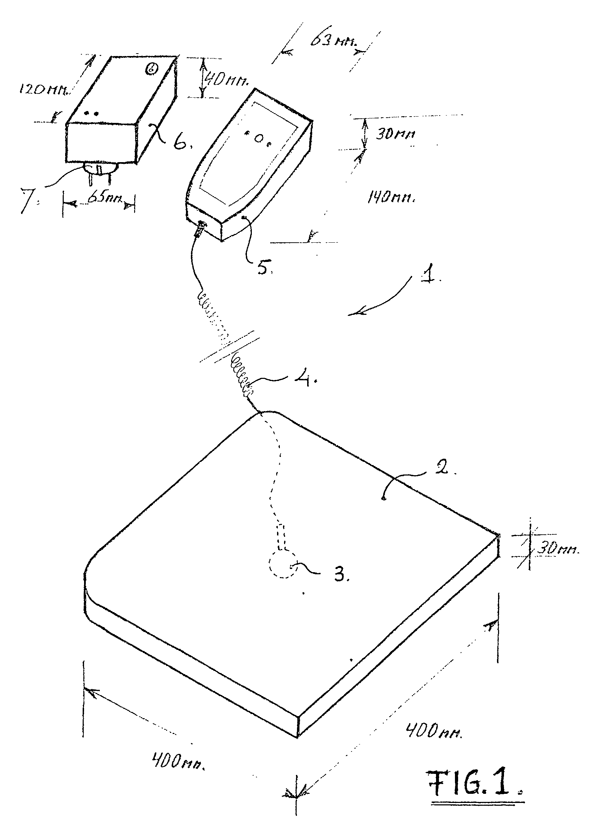

- Fig. 1: shows a view in oblique projection of the total detection system for the benefit of residents/patients of nursing homes according to a preferred embodiment of the invention, in which the main parts consist of a cushion or mat, a transmitter and a receiver;

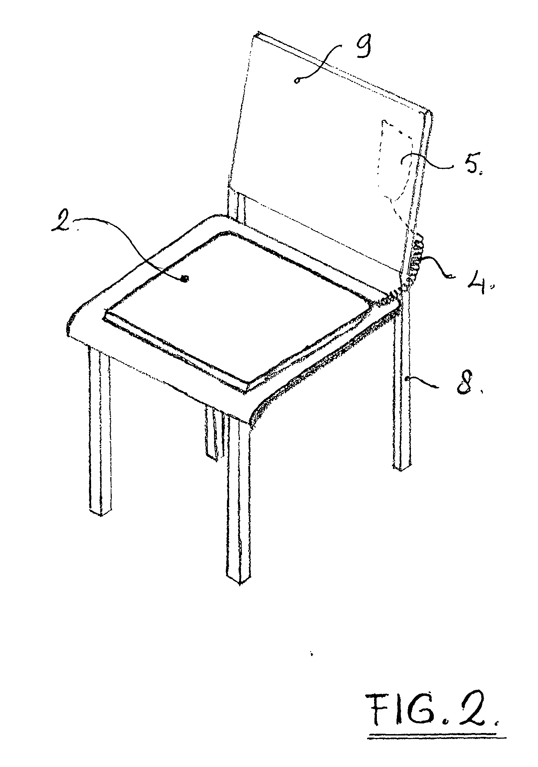

- Fig. 2: shows a view in oblique projection of a very simply constructed chair with on the seat the mentioned cushion or mat and the transmitter attached to the back of the chair;

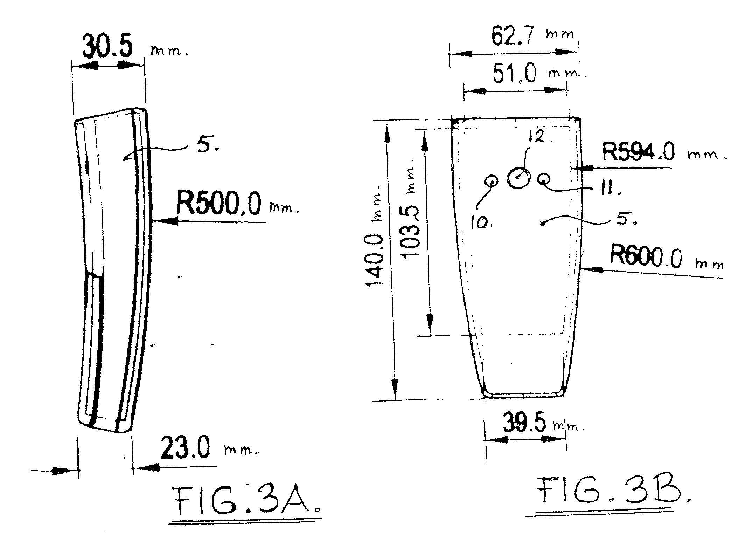

- Fig. 3A/3B: show a side and front view of the control or transmitter box according to a preferred embodiment;

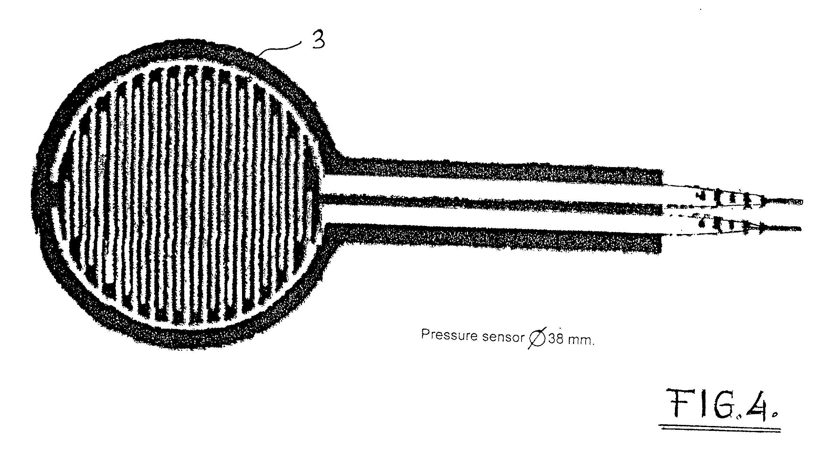

- Fig. 4: shows a front view of a suitable pressure sensor, placed in the cushion or mat of the detection system of the invention;
- Fig. 5: shows a typical connection between the applied load on the sensor and accompanying electrical resistance;

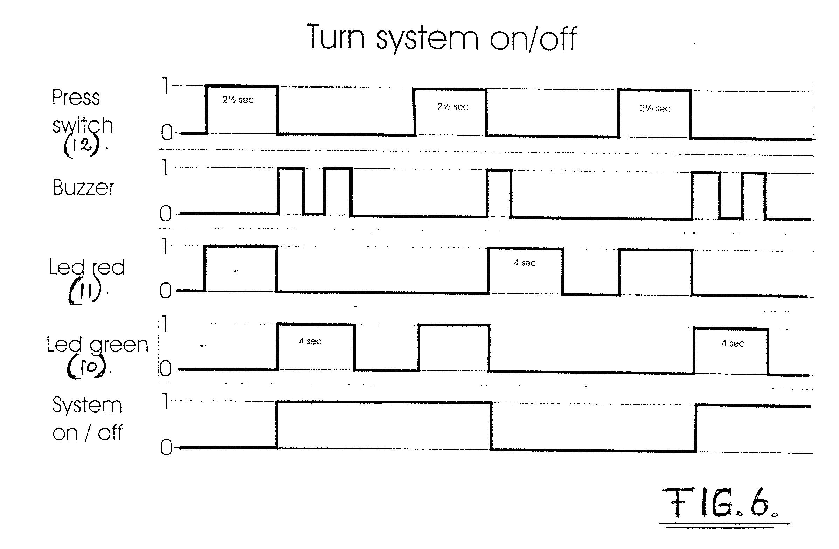

- Fig. 6: shows a schematic set-up of the operation of the electronic circuit for the on/off switch of the detection system according to the invention;

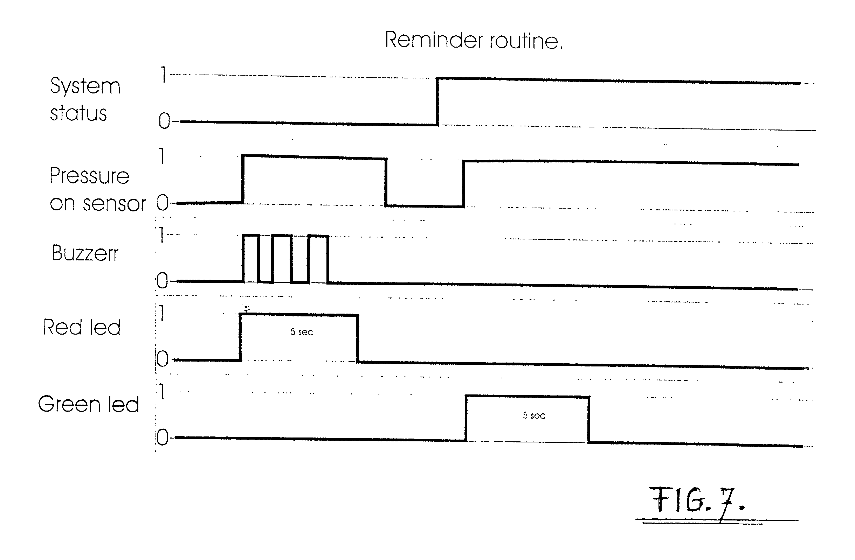

- Fig. 7: shows a schematic set-up of the operation of the electronic circuit for the reminder routine of the detection system according to the invention;

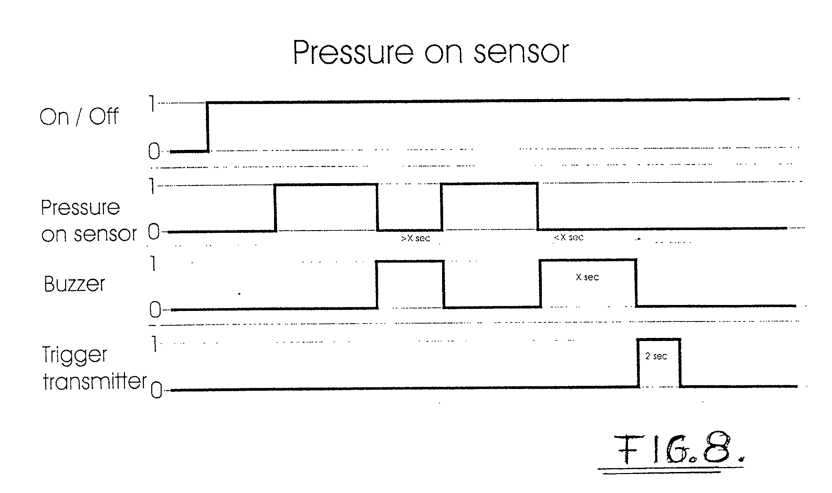

- Fig. 8: shows a schematic set-up of the operation of the electronic circuit if the sensor is loaded or unloaded by the force of the resident or patient;

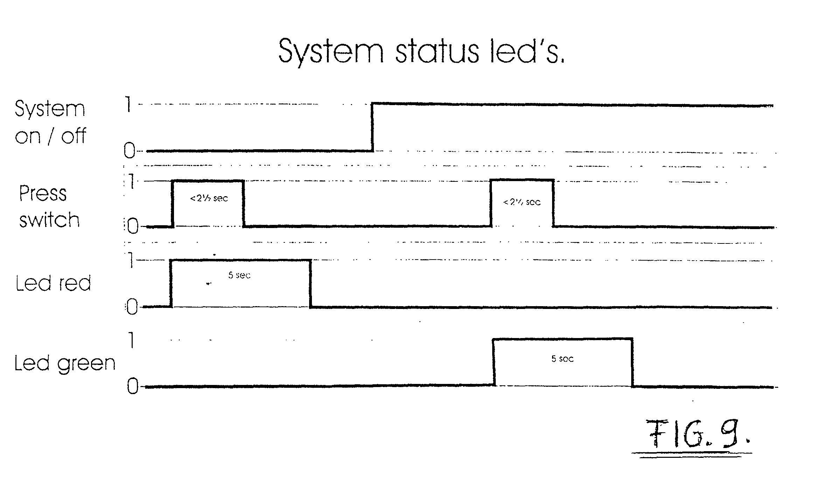

- Fig. 9: shows a schematic set-up of the operation of the electronic circuit for the status of the LEDs; and

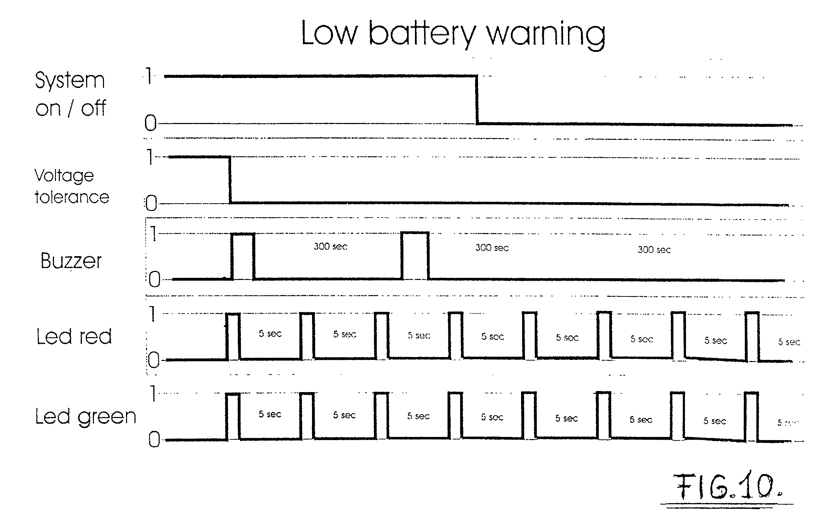

- Fig. 10: shows a schematic set-up of the operation of the electronic circuit for announcement of discharging batteries.

Figure 1 shows in oblique projection the detection system 1 which can be placed in a chair or bed for the benefit of nursing homes according to a preferred embodiment of the invention. The total detection system 1 consists of a cushion or mat 2 provided with a pressure sensor 3, which is connected to the control or transmitter box 5 by means of electronic wiring 4 for complete adjustment, reading and transmitting signals and such according to figures 6, 7, 8, 9 and 10. The mentioned signals can be transmitted further to the receiver 6 for calling a nurse. The preferred dimensions of the different parts of the total detection system are indicated in figure 1. The receiver 6 can be a common receiver, which can, for example, be energized by the lighting mains by means of a plug 7.

Figure 2 shows in oblique projection a simple chair 8 on which the cushion or mat 2 is placed and is connected with electric wires 4 to the control and transmitter box 5, that is attached on the outside of the back 9 of the chair for easy adjustment by the nurses.

Figures 3A and 3B show respectively a side and front view of the control or transmitter box 5. In the side view of figure 3A the control or transmitter box has a slightly bended shape to be adjusted to a general outer dimension of the back of a chair. In the front view of figure 3B the green LED 10 and red LED 11 or inverse indication is shown. By means of push a button 12 the detection system 1 is switched on. The operation and/or set-up of the adjustment of the on and off switching is indicated in figure 6. Here it is shown that the nurse should push the push button 12 for at least 2,5 seconds, after which a buzzer, the green LED 10 and the red LED 11, react accordingly. If someone else accidentally pushes the push button for less than 2,5 seconds, the system will not react. Arbitrarily 2,5 seconds have been chosen. Figure 9 shows the system with LED red and LED green.

Figure 4 shows a front view of a usable pressure sensor 3 with a round shape with a diameter of approximately 38 mm.

Figure 5 shows the connection between the weight force in Newton applied to the pressure sensor 3 and the accompanying resistance in kΩ. It is shown that particularly at a very slight pressure a large electronic change in resistance occurs. It concerns a FSR-sensor (Force Sensing Resistor). The curve drawn has been measured from experiments on a surface of 1 cm² at a pressure range of 0,1 to 100 Newton. Here, the electrical resistance value in Ohm varies from almost 0 to infinitely high.

Figure 7 schematically shows the operation of the remainder routine. This system of this routine works when, for example, the nurse has forgotten to push the button for at least 2,5 seconds and the resident/patient is already seated on the cushion or mat 2 (figure 1, 2). A buzzer is heard and the LED red lights up, for electric technical reasons, for 5 seconds. Until the status of the detection system 1 is switched on to position 1, the LED green will light up for 5 seconds.

Figure 8 shows that if the pressure on the pressure sensor is disconnected for more than X seconds and the patient has left the cushion or mat, a buzzer tone is given and through the transmitter box 5 a signal is given to a receiver 6 for 2 seconds to call a nurse. X seconds can be chosen arbitrarily.

Figure 10 shows the operation of the announcement of running down of the batteries of the transmitter box 5. The buzzer goes 2 times with interruptions of approximately 300 seconds and the red LED and green LED light up with interruptions of 5 seconds. The practical working of the system is as follows:
If a resident/patient, for example, sits down in a chair, the system indicates if it is on or not, which is of importance as a support for the nurses work. When the resident/patient stands up, the electronics in the cushion or mat 2 give an acoustic signal during a pre-set time (figure 8). The resident can react to this by sitting down again because he/she knows that he/she can fall. By using the behaviour of the detection system the patient corrects himself in some cases. If a patient, for example, does not react to this signal within 3 seconds, the transmitter box 5 will send a signal to the paging system or the receiver 6 coupled to a paging system or to an acoustic receiver system. The nurse or carer can now respond and can check and correct the person/resident/patient.

Finally it has to be emphasized, that the above description constitutes a preferred embodiment of the present invention and that further variations and modifications are still possible without departing the scope of this patent description.

## Claims

1. Apparatus constructed as a detection system shaped as a flat cushion or mat for placement on, in or underneath a chair's pillow or bed's mattress, in which the mentioned cushion or mat is provided with a mechanical/electronic total system for detecting and transmitting the applied change in load and status' on the mentioned cushion or mat in which the mentioned cushion or mat is mostly placed in a chair or bed of a resident, mostly an already admissioned patient in a home or nursing home, **characterized in that**, the mentioned mechanical/electronic total detection system (1) is constructed of at least one sensor (3) applied in the mentioned cushion or mat (2), which is mostly applied centrally in the mentioned cushion or mat (2), which sensor (3) is electrically or electronically coupled with a control or transmitter box (5) which is mounted elsewhere and which is supplied with electronic circuits for switching on and off of the mentioned total detection system (1), a reminder routine, an observation system for changes in pressure on the sensor (3) if this is forced by the patient and detection of rising and/or sitting down again or lying down of the patient, the status of the total detection system (1), the detection of almost running down of the battery and transmitting signals to the receiver box (6) for calling a nurse, and in which the mentioned receiver box (6), which can be a universal common receiver box (6), in which the mentioned mechanical/electronic total detection system (1) is energized by a battery with an operating voltage of 3 Volt, in which for the contents and the exterior of the mentioned cushion or mat (2) can be provided with comfort increasing facilities.

2. Apparatus as claimed in claim 1, **characterized in that**, the mentioned sensor (3) is a pressure sensor of the FSR-sensor type (Force Sensing Resistor).

3. Apparatus as claimed in claim 2, **characterized in that**, the mentioned FSR-sensor is built-up in three layers, being a load-bearing foil with pressed-on FSR-layer, and adhesive layer (spacer) and a load-bearing foil with electrodes, in which this can have the shape of a single element with a diameter of approximately 40 mm, in which the connection between the applied forces in Newton on the sensor (3) and the electronic resistance R in kΩ can be as in figure 5.

4. Apparatus as claimed in claim 1, **characterized in that**, the set-up and operation of the electronic circuit and/or programming for switching on and off is indicated in figure 6.

5. Apparatus as claimed in aforementioned claims, **characterized in that**, the set-up and operation of the electronic circuit for the reminder routine is indicated in figure 7.

6. Apparatus as claimed in aforementioned claims, **characterized in that**, the set-up and operation of the electronic circuit and/or programming for sitting/lying down and getting up is indicated in figure 8.

7. Apparatus as claimed in aforementioned claims, **characterized in that,** the set-up and operation of the electronic circuit and/or programming for the LED's status is indicated in figure 9.

8. Apparatus as claimed in aforementioned claims, **characterized in that,** the set-up and operation of the electronic circuit and/or programming for battery discharge announcement is indicated in figure 10.

9. Apparatus as claimed in claim 1, **characterized in that**, the mentioned control or transmitter box (5) provided with the mentioned electronic circuits can roughly have dimensions of 140 x 65 x 30 mm and operating and report possibilities can be buttons and displays such as on/off, and adjustment of an alarm deceleration, battery discharge indication, transmitter (time, quantity of beeps) in which the on switch (12) must be pushed in at least 2,5 seconds in order to separate deliberately switching on from accidentally touching the on button.

10. Apparatus as claimed in claim 1, **characterized in that**, the mentioned outer dimensions of the mentioned cushion or mat with L x W x H can be approximately 400 x 400 x 30 mm.

11. Apparatus as claimed in claims 1 and 10, **characterized in that**, the contents of the mentioned cushion or mat (2) with regard to flexibility is made of the material BULTEX, being a durable resilient material.

12. Apparatus as claimed in claims 1, 10 and 11, **characterized in that**, the upholstery of the mentioned cushion or mat (2) for increasing comfort with regard to water tightness and antiskid is made of so called incontinent material.

13. Apparatus as claimed in claims 1, 4, 5, 6, 7 and 8, **characterized in that,** the check for switched-on battery can be done by an indicator light and that this is only temporary, for example, a few seconds, in order to save the battery.

14. Apparatus as claimed in claim 1, **characterized in that**, the geometric distance between the control or transmitter box (5) mounted elsewhere and the mentioned receiver box (6) of carers or nurses is approximately 50 m.

15. Apparatus as claimed in claims 1 and 14, **characterized in that**, maximally 10 cushions or mats (2) can be read in on the mentioned receiver box (6), so that in each accommodation a closed surveillance/monitoring system for the resident and/or patient is created.

16. Apparatus as claimed in claim 15, **characterized in that**, the mentioned control or transmitter box (5) with matching cushion in the accommodation for the residents/patients of the home or nursing home is specifically read in to the mentioned control or transmitter box (5).
